# EUROPEAN PATENT APPLICATION

(11) **EP 4 249 604 A2**
(43) Date of publication of application: **27.09.2023**
(21) Application number: 23175302.1
(22) Date of filing: 22.07.2016
(51) Int. Cl.: C12Q 1/6806

(54) **COMPOSITIONS AND METHODS OF RNA ANALYSIS**

(30) Priority: 24.07.2015 US 201562196725 P
(62) Divisional of application: 16831111.6
(71) Applicant: The Johns Hopkins University, Baltimore, MD 21201 (US)
(72) Inventor: CREDLE, Joel, Gaithersburgh, MD 20878 (US); LARMAN, Harry, Benjamin, Baltimore, MD 21224 (US)
(74) Representative: Murgitroyd & Company

(57) **Abstract**

The present disclosure relates to compositions and methods of RNA analysis. In particular, the present disclosure provides a method of RNA analysis that includes obtaining a sample, applying one or more multi-partite probes to the sample, where each of the one or more multi-partite probes includes at least two sub-probes, annealing at least one of the applied one or more multi-partite probes to at least one target nucleic acid within the sample, and ligating the at least two sub-probes associated with the at least one annealed multi-partite probe to create a target nucleic acid proxy that can be detected.

## Description

### RELATED APPLICATIONS

This application is an International Patent Application which claims the benefit of priority under 35 U.S.C. § 119(e) to U.S. Provisional Application No: 62/196,725, filed on July 24, 2015 and entitled, "Compositions and Methods of RNA Analysis", which is incorporated herein by reference in its entirety.

### BACKGROUND OF THE INVENTION

Precision medicine relies upon the ability of researchers and pathologists to molecularly characterize patient specimens, including resected tissues, e.g., tumor tissues, and biopsy materials. The development of advanced techniques for DNA analysis, such as high throughput DNA sequencing (or next generation DNA sequencing, NGS), has contributed greatly to the detection of genetic lesions associated with cancer. However, the ability to characterize gene expression profiles in fixed tissue specimens (e.g. formalin fixed paraffin embedded tissue sections) has lagged behind because the fixation process degrades the quality of RNA in the sample, which significantly inhibits the ability to extract important information from the sample. Since information about gene expression patterns in such fixed tissue specimens is of great value, there is an unmet need for a platform that enables robust analysis of the RNA within fixed tissue specimens.

### SUMMARY OF THE INVENTION

The present disclosure provides compositions and methods for analyzing the presence and expression level of nucleic acids (e.g., RNA) in a sample (e.g., a cell, an organ, a tissue, and the like).

In one aspect, the disclosure provides a method involving obtaining a sample, applying one or more multi-partite probes to the sample, where each of the one or more multi-partite probes includes at least two sub-probes, annealing at least one of the applied one or more multipartite probes to at least one target nucleic acid within the sample, and ligating the at least two sub-probes associated with the at least one annealed multi-partite probe to create a target nucleic acid proxy that can be detected. The method may further comprise releasing the target nucleic acid proxy from the target nucleic acid and amplifying the target nucleic acid proxy. In some cases, each probe comprises an oligonucleotide, e.g., DNA, RNA, or a mixture of both DNA and RNA. Preferably, the target nucleic acid is RNA. In some cases, the target nucleic acid proxy is modified.

In one aspect, the sub-probes comprise appended primer binding site which facilitate the post-ligation amplification of the target nucleic acid proxy. In some aspects, each sub-probe comprises an oligonucleotide. In other aspects, the sub-probes (which may also be referred to as "acceptor sub-probes") have a 3'-termination of at least two RNA bases. In one aspect, the sub-probe is a DNA oligonucleotide that has a 3'-termination of at least two RNA bases. In another aspect, the sub-probe is a DNA oligonucleotide that has a 5'-phosphorylation.

In an embodiment, the at least two sub-probes may be ligated with an enzyme, a chemical reaction, or a photoreaction. The enzyme may be a ligase. In particular embodiments, the enzyme may be one of the following ligases: a T4 RNA Ligase 2 (Rnl2), T4 DNA Ligase, a Chlorella virus DNA Ligase (PBCV-1 DNA Ligase), a Rnl2 derivative, PBCV-1 derivative, or any combination thereof.

In an embodiment, the sample may be selected from the group consisting of a cell, an organ, a tissue, and any combination thereof. In some examples, the sample may be fixed using non-formalin reagents, including, for example, glutaraldehyde, mercurial, oxidizing agents, alcohols, and picrates. In other examples, the sample may include fixed cells in suspension or a fixed tissue culture. The sample may be obtained in a highly degraded form prior to fixation. In particular, the sample may be a formalin fixed paraffin embedded (FFPE) tissue sample.

In an embodiment, the target nucleic acid proxy may be released from the target nucleic acid by an endonuclease or recovered by denaturing the target nucleic acid proxy containing the at least two ligated sub-probes. The endonuclease may be RNaseH, RNaseA, RNase If, or RNaseHIII.

In an embodiment, the amplified target nucleic acid proxy may be analyzed using a technique such as, for example, Next Generation Sequencing (NGS), Deep Sequencing, mass spectrometry based sequence or length analysis, DNA fragment sequence or length analysis by gel electrophoresis or capillary electrophoresis, and/or hybridization on immobilized detection probes.

In an embodiment, the fixed tissue specimen may be processed into sections having a thickness of 1-100 microns, e.g., 1micron, 5 microns, 10 microns, 15 microns, 20 microns, 25 microns, 30 microns, 35 microns, 40 microns, 45 microns, 50 microns, 60 microns, 65 microns, 70 microns, 75 microns, 80 microns, 85 microns, 90 microns, 95 microns, or 100 microns.

In an embodiment, the at least two sub-probes of the plurality of multi-partite probes may be about 10-200 nucleotides in length, e.g., about 10, about 15, about 20, about 25, about 30, about 35, about 40, about 50, about 75, about 100, about 125, about 150, about 175, or about 200 nucleotides in length. In another embodiment, each of the at least two sub-probes of the plurality of multi-partite probes may be about 15-30 nucleotides in length, e.g., about 15, about 16, about 17, about 18, about 19, about 20, about 21, about 22, about 23, about 24, about 25, about 26, about 27, about 28, about 29, or about 30 nucleotides in length.

In an embodiment, the nucleic acid proxy may be amplified using PCR. In an embodiment, the PCR includes about 20-50 cycles, e.g., about 20, about 25, about 30, about 35, about 40, about 45, or about 50 cycles.

In an embodiment, each of the plurality of multi-partite probes includes two sub-probes. In a related embodiment, each of the plurality of multi-partite probes includes three sub-probes.

Optionally, the above-mentioned method is performed sequentially on a sample, i.e., the method is performed twice, three times, four times, five times, six times, seven times, eight times, nine times, or ten times on the same sample. In some cases, the specimen (i.e., sample) is archived between sequential assays.

The methods described herein are also useful for diagnosing infection, e.g., viral infection, bacterial infection, or fungal infection. For example, the sample is obtained from a subject suspected of or at risk of developing a viral infection, a bacterial infection, or a fungal infection. Optionally, the target nucleic acid is selected from the group consisting of a viral nucleic acid, a bacterial nucleic acid, and a fungal nucleic acid. In some cases, the method further comprises releasing the target nucleic acid proxy from the target nucleic acid; amplifying the target nucleic acid proxy; and sequencing the target nucleic acid proxy, thereby identifying a viral nucleic acid, a bacterial nucleic acid or a fungal nucleic acid and diagnosing a viral infection, a bacterial infection, or a fungal infection, respectively, in the subject. Preferably, after diagnosis, the subject is treated with an anti-fungal agent, an anti-bacterial agent, or an antiviral agent.

Exemplary fungal infections may include infections derived from the following organisms: *A cremonium* sp., *Aspergillus clavatus, Aspergillus flavus, Aspergillus fumigatus, Aspergillus glaucus, Aspergillus nidulans, Aspergillus niger, Aspergillus ochraceus, Aspergillus terreus, Aspergillus unguis, Aspergillus ustus, Beauveήa* sp., *Bipolaris* sp., *Blastoschizomyces* sp., *Blastomyces dermatitidis, Candida albicans, Candida glabrata, Candida guilliermondii, Candida kefyr, Candida krusei, Candida lusitaniae, Candida parapsilosis, Candida tropicalis, Chrysosporium* sp., *Cladosporium* sp., *Coccidioides immitis, Cryptococcus neoformans var gattii* serotype B, *Cryptococcus neoformans* serotype A, *Cryptococcus laurentii, Cryptococcus terreus, Curvularia* sp., *Fusarium* sp., *Filobasidium capsuligenum, Filobasidiella* (Cryptococcus) *neoformans var bacillispora* serotype C, *Filobasidiella* (Cryptococcus) *neoformans var neoformans* serotype D, *Filobasidium uniguttulatum, Geotrichum* sp., *Histoplasma capsulatum, Malbranchea* sp., *Mucor* sp., *Paecilomyces* sp., *Para coccidioides brasiliensis, Penicillium* species, *Pneumocystis carinii, Pseudallescheria boydii, Rhizopus* sp., *Sporothrix schenkii, Scopulariopsis brevicaulis* sp., *Scopulariopsis brumpti, Saccharomyces cerevisiae,* and *Trichosporon beigelii.*

Exemplary bacterial infections may include infections derived from the following organisms: *Bacillus anthracis, Bordetella pertussis, Borrelia burgdorferi, Brucella abortus, Brucella canis, Brucella melitensis, Brucella suis Brucellosis, Campylobacter jejuni, Chlamydia pneumoniae* respiratory infection, *Chlamydia psittaci, Chlamydia trachomatis, Lymphogranuloma venereum, Clostridium botulinum, Clostridium difficile, Clostridium perfringens, Clostridium tetani, Corynebacterium diphtheria, Enterococcus faecalis, Enterococcus faecium, Escherichia coli, Francisella tularensis, Haemophilus influenza, Helicobacter pylori, Legionella pneumophila, Leptospira interrogans, Listeria monocytogenes, Mycobacterium leprae, Mycobacterium tuberculosis, Mycoplasma pneumonia, Neisseria gonorrhoeae, Neisseria meningitides, Pseudomonas aeruginosa, Rickettsia rickettsia, Salmonella typhi, Salmonella typhimurium, Shigella sonnei, Staphylococcus aureusa, Staphylococcus epidermidis, Staphylococcus saprophyticus, Streptococcus agalactiae, Streptococcus pneumonia, Streptococcus pyogenes, Treponema pallidum, Vibrio cholera, Yersinia pestis.*

Exemplary viral infections may include infections derived from the following organisms: *Adenoviruses, Avian influenza, Influenza* virus type A, *Influenza* virus type B, Measles, *Parainfluenza* virus, *Respiratory syncytial virus* (RSV), *Rhinoviruses, SARS-CoV* (*Severe* Acute Respiratory Syndrome), *Coxsackie* viruses, *Enteroviruses, Poliovirus, Rotavirus, Hepatitis B* virus, *Hepatitis C* virus, *Bovine viral diarrhea virus* (surrogate), *Herpes simplex 1, Herpes simplex 2, Human cytomegalovirus, Varicella zoster virus, Human immunodeficiency virus 1* (HIV-1), *Human immunodeficiency virus 2* (HIV-2), *Simian immunodeficiency virus* (SIV), *Simian human immunodeficiency virus* (SHIV), *Dengue virus, Hantavirus,* Hemorrhagic fever viruses, *Lymphocyti, choromeningitis* virus, Smallpox virus surrogates (Cowpox, Monkeypox, Rabbitpox), Vaccinia virus, Venezuelan equine encephalomyelitis virus (VEE), West Nile virus, Yellow fever virus, Zika virus.

In one aspect, the disclosure provides a method involving obtaining a fixed tissue sample from a subject, applying one or more multi-partite probes to the fixed tissue sample, where each of the one or more multi-partite probes includes at least two sub-probes, annealing at least one of the one or more applied multi-partite probes to a target nucleic acid within the fixed tissue sample, ligating the at least two sub-probes associated with the at least one annealed multi-partite probe to create a target nucleic acid proxy, and stamping the ligated probes onto a replica surface coated with a plurality of immobilized PCR primers.

In an embodiment, the at least two sub-probes may be ligated with an enzyme, a chemical reaction, or a photoreaction. In an embodiment, the enzyme may be a ligase. In particular, the ligase may be a T4 RNA Ligase 2 (Rnl2), T4 DNA ligase, a Chlorella virus DNA Ligase (PBCV-1 DNA Ligase), a Rnl2 derivative, PBCV-1 derivative, or any combination thereof.

In an embodiment, the sample is a formalin fixed paraffin embedded (FFPE) tissue sample.

In an embodiment, the target nucleic acid proxy is subjected to solid-phase amplification. In an embodiment, the solid-phase PCR amplification may be Bridge PCR or rolling circle amplification (RCA). In an embodiment, the target surface comprises a polymer-coated glass microscope slides containing immobilized PCR primers.

In an embodiment, the tissue sample comprises resected tumor tissues or biopsy materials.

In another embodiment, the disclosure provides a method including obtaining a fixed tissue sample from a subject, applying one or more multi-partite probes to the fixed tissue sample, wherein each of the one or more multi-partite probes includes at least two sub-probes; annealing at least one of the one or more applied multi-partite probes to a target nucleic acid within the fixed tissue sample; ligating the at least two sub-probes associated with the at least one annealed multi-partite probe to create a target nucleic acid proxy within the fixed tissue sample; and stamping the ligated probes onto a replica surface coated with a plurality of immobilized PCR primers. In embodiments, this method may be referred to as the "stamping approach" or "stamping method."

In one aspect, the sub-probes are ligated with an enzyme, a chemical reaction, or a photoreaction. In some examples, the enzyme may be a ligase selected from the group consisting of a T4 RNA Ligase 2 (Rnl2), T4 DNA Ligase, a Chlorella virus DNA Ligase (PBCV-1 DNA Ligase), a Rnl2 derivative, PBCV-1 derivative, and any combination thereof.

In one aspect, the stamping method described above may use a formalin fixed paraffin embedded (FFPE) tissue sample, resected tumor tissues or biopsy materials. In other embodiments, the target nucleic acid proxy is subjected to solid-phase amplification, including, for example, Bridge PCR or rolling circle amplification (RCA), including a polymer-coated glass microscope slides containing immobilized Bridge PCR primers.

### Definitions

By "agent" is meant any small compound, antibody, nucleic acid molecule, or polypeptide, or fragments thereof.

By "alteration" is meant a change (increase or decrease) in the expression levels or activity of a gene or polypeptide as detected by standard art known methods such as those described herein. As used herein, an alteration includes a 10% change in expression levels, preferably a 25% change, more preferably a 40% change, and most preferably a 50% or greater change in expression levels.

"Bridge PCR" is a method of localized, solid-phase PCR amplification, in which DNA fragments are amplified by primers attached to a surface (rather than in solution), thus forming surface-immobilized DNA clusters. If the clusters are sufficiently separated, they can be clonally sequenced.

In this disclosure, "comprises," "comprising," "containing", and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Detect" refers to identifying the presence, absence, or amount of the nucleic acid (e.g., RNA) to be detected.

By "detectable label" is meant a composition that when linked to a molecule of interest renders the latter detectable, via, for example, spectroscopic, photochemical, biochemical, immunochemical, or chemical means. For example, useful labels may include radioactive isotopes, magnetic beads, metallic beads, colloidal particles, fluorescent dyes, electron-dense reagents, enzymes (for example, as commonly used in an ELISA), biotin, digoxigenin, or haptens.

By "effective amount" is meant the amount of an agent required to ameliorate the symptoms of a disease relative to an untreated patient. The effective amount of active agent(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "FFPE" is meant formalin fixed paraffin embedded tissue. FFPE samples are derived from tissues (often suspected tumor samples) that are fixed with formalin to preserve structuralspatial and biomolecule characteristics (e.g., cytoskeletal and protein structure) and then embedded in a type of paraffin wax so the tissue can be sliced, typically 5-10 microns thick. Formalin irreversibly cross-links proteins via the amino groups thus preserving the structural integrity of the cells so they can be stained with dyes or with immunostains used to analyze for abnormalities in the tissue that indicate altered cellular conditions, e.g., cancer. However, the effect of these cross-linking fixatives on the RNA and DNA nucleic acids within the sample is detrimental to the sensitivity and specificity achievable in current molecular assays e.g., molecular assays which use DNA or RNA derived from FFPE samples. Additionally, samples may be prepared using non-formalin reagents, including, for example, glutaraldehyde, mercurial, oxidizing agents, alcohols, and picrates.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, 100, 200, 300, 400, 500, 600, 700, 800, 900, or 1000 nucleotides or amino acids.

"Hybridization" means hydrogen bonding, which may be Watson-Crick, Hoogsteen or reversed Hoogsteen hydrogen bonding, between complementary nucleobases. For example, adenine and thymine are complementary nucleobases that pair through the formation of hydrogen bonds.

By "infection" is meant the invasion of an organism's body by disease-causing agents, their multiplication and the reaction of the host to these organisms and the toxins they produce. The infection may be caused by any microbes/microorganisms, including for example, bacteria, fungi, and viruses. Microorganisms can include all bacterial, archaean, and the protozoan species. This group also contains some species of fungi, algae, and certain animals. In some embodiments, viruses may be also classified as microorganisms.

By "isolated polynucleotide" is meant a nucleic acid (e.g., RNA, DNA, cDNA, etc.) that is free of the genes which, in the naturally-occurring genome of the organism from which the nucleic acid molecule of the invention is derived, flank the gene. The term therefore includes, for example, a recombinant DNA that is incorporated into a vector; into an autonomously replicating plasmid or virus; or into the genomic DNA of a prokaryote or eukaryote; or that exists as a separate molecule (for example, a cDNA or a genomic or cDNA fragment produced by PCR or restriction endonuclease digestion) independent of other sequences. In addition, the term includes an RNA molecule that is transcribed from a DNA molecule, as well as a recombinant DNA that is part of a hybrid gene encoding additional polypeptide sequence.

"Laser capture microdissection" or "LCM" is a method for isolating specific cells from microscopic regions of tissues, cells or organisms. LCM is a method to procure subpopulations of tissue cells under direct microscopic visualization. LCM technology can harvest the cells of interest directly or can isolate specific cells by cutting away unwanted cells to give histologically pure enriched cell populations.

Ligation in situ Hybridization or "LISH" is a methodology disclosed herein for multiplexed measurement of gene expression, suited for analysis of fixed tissue specimens. One embodiment of LISH utilizes the T4 RNA Ligase 2 (Rnl2) and chimeric DNA-RNA hybridization probes, which when annealed, then become ligated, where they serve as faithful proxies to a respective target's expression level.

"LISH-stAmp" is a method to stamp and Amplify LISH product on a target surface to preserve spatial information.

By "marker" is meant any protein or polynucleotide having an alteration in expression level or activity that is associated with a disease or disorder.

By "modulate" is meant alter (increase or decrease). Such alterations are detected by standard art known methods such as those described herein.

By "neoplasia" is meant a disease or disorder characterized by excess proliferation or reduced apoptosis. Illustrative neoplasms for which the invention may be used include, but are not limited to pancreatic cancer, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, glioblastoma multiforme, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma).

By "NGS" is meant Next Generation Sequencing. NGS platforms perform massively parallel sequencing, during which millions of fragments of DNA from a single sample are sequenced in unison. Massively parallel sequencing technology facilitates high-throughput sequencing, which allows an entire genome to be sequenced in less than one day. The creation of NGS platforms has made sequencing accessible to more labs, rapidly increasing the amount of research and clinical diagnostics being performed with nucleic acid sequencing.

Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a single or double-stranded nucleic acid molecule. Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a single or double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30°C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 µg/ml denatured salmon sperm DNA (ssDNA). In a most preferred embodiment, hybridization will occur at 42°C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred embodiment, wash steps will occur at 25°C in 30 mM NaCl, 3 mM trisodium citrate, and 0.1% SDS. In a more preferred embodiment, wash steps will occur at 42°C in 15 mM NaCl, 1.5 mM trisodium citrate, and 0.1% SDS. In a more preferred embodiment, wash steps will occur at 68°C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

"Sequencing" or any grammatical equivalent as used herein may refer to a method used to sequence the amplified target nucleic acid proxy. The sequencing technique may include, for example, Next Generation Sequencing (NGS), Deep Sequencing, mass spectrometry based sequence or length analysis, or DNA fragment sequence or length analysis by gel electrophoresis or capillary electrophoresis. Compatible sequencing techniques may be used including singlemolecule real-time sequencing (Pacific Biosciences), Ion semiconductor (Ion Torrent sequencing), pyrosequencing (454), sequencing by synthesis (Illumina), sequencing by ligation (SOLiD sequencing), chain termination (Sanger sequencing), Nanopore DNA sequencing (Oxford Nanosciences Technologies), Heliscope single molecule sequencing (Helioscope inc), sequencing with mass spectrometry, DNA nanoball sequencing, sequencing by hybridization, and tunnelling currents DNA sequencing.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e-3 and e-100 indicating a closely related sequence.

"Primer set" means a set of oligonucleotides that may be used, for example, for PCR. A primer set would consist of at least 2, 4, 6, 8, 10, 12, 14, 16, 18, 20, 30, 40, 50, 60, 80, 100, 200, 250, 300, 400, 500, 600, or more primers.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 10 in the other direction.

By "reduces" is meant a negative alteration of at least 10%, 25%, 50%, 75%, or 100%.

By "reference" is meant a standard or control condition.

A "reference sequence" is a defined sequence used as a basis for sequence comparison or a gene expression comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 40 nucleotides, preferably at least about 60 nucleotides, more preferably at least about 75 nucleotides, and even more preferably about 100 nucleotides or about 300 or about 500 nucleotides or any integer thereabout or therebetween.

As used herein, "obtaining" as in "obtaining an agent" includes synthesizing, purchasing, or otherwise acquiring the agent.

By "subject" is meant a mammal, including, but not limited to, a human or non-human mammal, such as a bovine, equine, canine, ovine, or feline.

As used herein, the term "sub-probe" may refer to any of the two or more probes that bind the contiguous target sequence without leaving any unbound intervening nucleotides. In some embodiments, the multi-partite probe described herein may include at least two "sub-probes." In another embodiment, each of the at least two sub-probes of the plurality of multipartite probes may be about 10-50 nucleotides in length. Once the probes are ligated, the ligated multi-partite probe (alternatively, the "ligated sub-probe") may be released from the RNA. In some embodiments, the sub-probe may contain appended primer binding site (e.g., adapters) to facilitate subsequent amplification of the target nucleic acid proxy. In other embodiments, at least one of the two or more sub-probes may be referred to as "acceptor sub-probes" that have a 3'-termination of at least two RNA bases.

As used herein, "appended primer binding" sites may refer to binding sites within the multi-partite probe or sub-probes described herein that facilitate amplification of the target nucleic acid proxy. "Appended primer binding sites" may also be referred to as "adapters."

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

As used herein, the terms "prevent," "preventing," "prevention," "prophylactic treatment" and the like refer to reducing the probability of developing a disorder or condition in a subject, who does not have, but is at risk of or susceptible to developing a disorder or condition.

Unless specifically stated or obvious from context, as used herein, the term "or" is understood to be inclusive. Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

Other features and advantages of the invention will be apparent from the following description of the preferred embodiments thereof, and from the claims. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All published foreign patents and patent applications cited herein are incorporated herein by reference. Genbank and NCBI submissions indicated by accession number cited herein are incorporated herein by reference. All other published references, documents, manuscripts and scientific literature cited herein are incorporated herein by reference. In the case of conflict, the present specification, including definitions, will control. In addition, the materials, methods, and examples are illustrative only and not intended to be limiting.

### BRIEF DESCRIPTION OF THE DRAWINGS

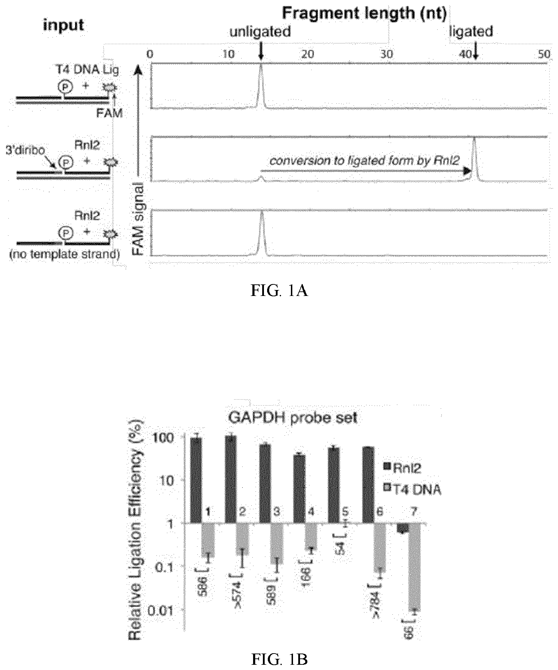

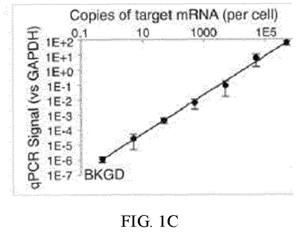

Figure 1A-Figure 1C depicts an electropherogram, a bar chart and a graph respectively. Figure 1A shows a schematic showing a capillary electropherogram after enzymatic ligation. RNA bases are colored grey, DNA bases are colored black and FAM is the fluorescent label. T4 DNA Ligase does not produce measurable ligation product (top). Rnl2 (T4 RNA ligase 2) ligates the chimeric DNA-RNA probe set in the presence of the RNA template strand (middle), but not in its absence (bottom). Figure 1B depicts a bar graph showing that seven unique RASL (RNA Annealing, Selection and Ligation sequencing) probe sets targeting GAPDH (glyceraldehyde 3-phosphate dehydrogenase) mRNA were evaluated on cellular RNA. The fold difference in ligation efficiency of Rnl2 vs. T4 DNA Ligase is shown for each probe set. Figure 1C depicts a graph showing the signal from an Rnl2-based RASL assay designed to measure a spike-in RNA sequence in a constant background of cellular RNA. Background level (BKGD) of SYBR qPCR assay.

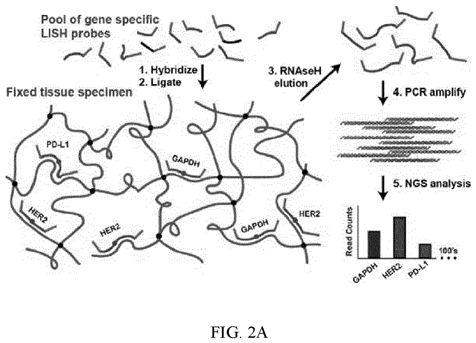

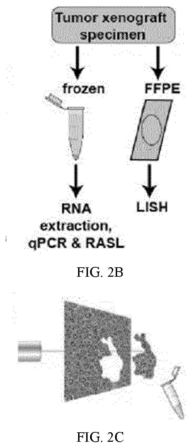

Figure 2A-Figure 2C depict a diagram, a process chart, and an image, respectively. Figure 2A shows a process chart depicting ligation in situ hybridization sequencing (LISH-seq). A pool of gene specific probe sets is hybridized to their target RNA in the fixed tissue. After washing away excess probe, Rnl2 ligates adjacently annealed probes. Ligation product can then be recovered, amplified and sequence-analyzed. Figure 2B depicts a cartoon depicting initial experiments that compare transcript quantification by RT-qPCR, RASL and LISH using both frozen and FFPE (formalin fixed paraffin embedded) tissue specimens as described. Figure 2C is an image depicting the utility of LISH-seq for analysis of microdissected tissues.

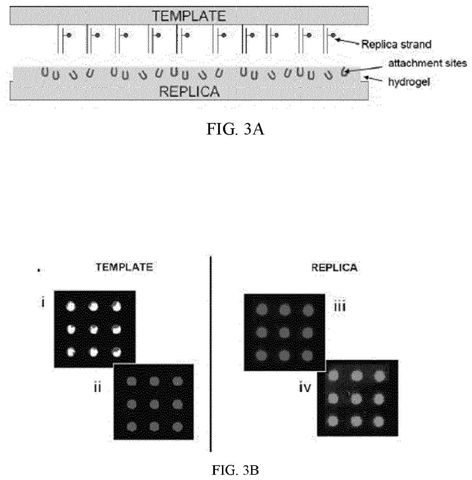

Figure 3A-Figure 3B show a template diagram and a template/replica image, respectively. Figure 3A shows a template diagram depicting stamping of DNA to target surface. A template for ssDNA microarray that is used to guide delivery of biotinylated cDNA onto a streptavidin-incorporated hydrogel film-coated replica surface is shown. Figure 3B is an image depicting Cy5/biotin-labeled cDNA that was transferred to a replica surface. (i) shows template pre-transfer, (ii) shows post-transfer. The replica surface is imaged (iii) pre-hybridization and (iv) post-hybridzation with a Cy3 complementary sequence. Spots are ~100 microns.

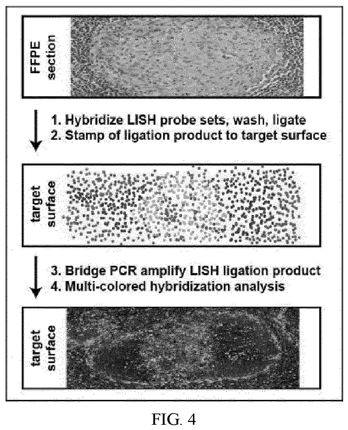

Figure 4 depicts a LISH-stAmp probe analysis of a formalin fixed paraffin embedded (FFPE) sample according to an exemplary embodiment of the disclosure. The top image depicts the LISH-stAmp process. LISH probe hybridization and ligation would be performed on the FFPE section. The middle image depicts stamping of ligation product onto the bridge PCR target surface (each dot represents a single molecule of ligation product). The bottom image depicts bridge PCR, which would amplify the LISH signal in situ for subsequent analysis using panels of hybridization probes.

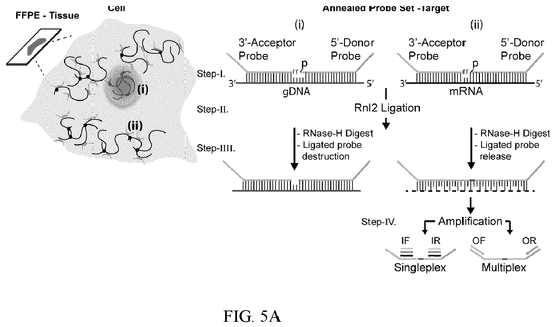

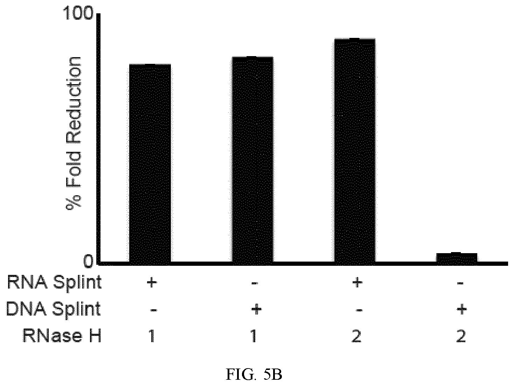

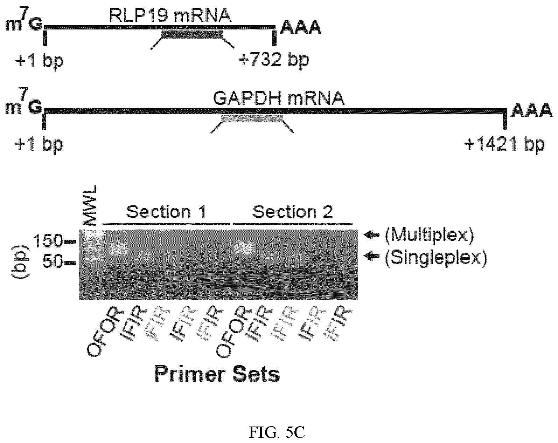

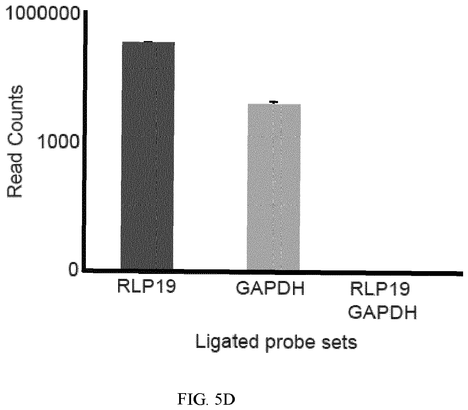

Figure 5A-Figure 5D depict a diagram, a bar graph, a gel, and a bar graph, respectively. Figure 5A depicts a diagram describing an approach for efficiently measuring many RNA sequences in FFPE specimens without RNA purification or reverse transcription. Figure 5B is a bar graph that indicates that DNA-annealed diribonucleotide-containing DNA sequences were cleaved by RNAse H2, whereas RNA-annealed sequences were not. Figure 5C is a gel that indicates that FFPE RNA could serve as a suitable template for Rnl2-mediated probe ligation *in situ.* Figure 5D is a bar graph indicating that the end-point PCR analysis of the RNAse H recovered ligation product by next generation DNA sequencing revealed the correct probe pairs were indeed ligated *in situ,* whereas the mismatched probe sets were not ligated at significant levels.

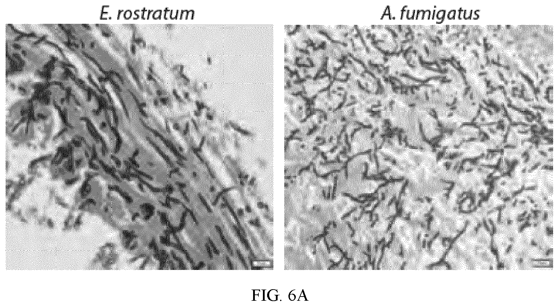

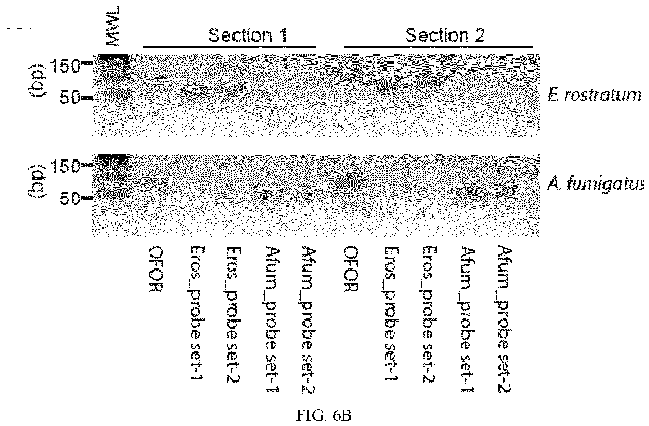

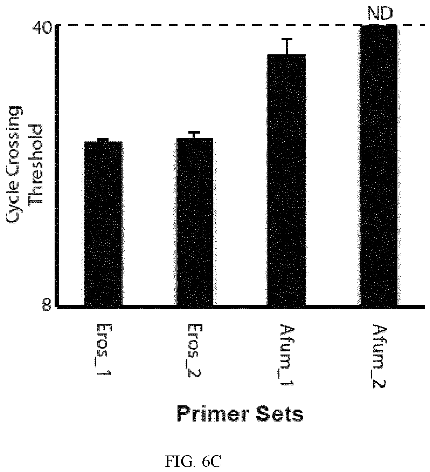

Figures 6A-6C depict biopsy images, a gel, and a bar graph, respectively indicating that LISH can be used to diagnose infection using preserved clinical FFPE specimens. Figure 6A depicts biopsies of brain tissue infected with *Exserohilum rostratum* (left) and *Aspergillus fumigatus* (right). Figure 6B depicts a gel indicating that *Exserohilum rostratum* detector probes reported strong on-target signal from the index patient, but not from biopsies of uninfected tissues or sections containing unrelated infections, such as the closely *related Aspergillus fumigatus.* Figure 6C is a bar graph indicating that *Exserohilum rostratum* detector probes reported strong on-target signal from the index patient, but not from biopsies of uninfected tissues or sections containing unrelated infections, such as the closely *related Aspergillus fumigatus.*

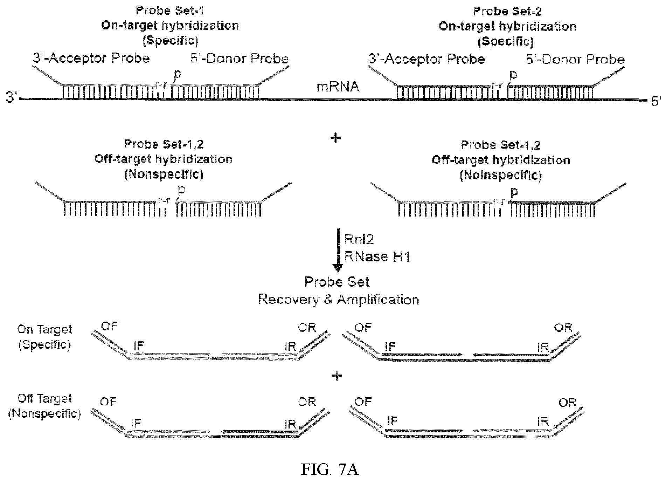

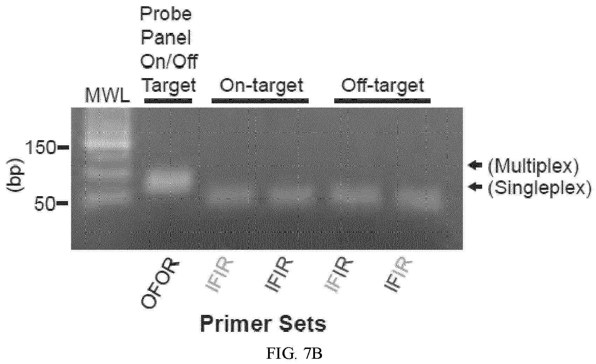

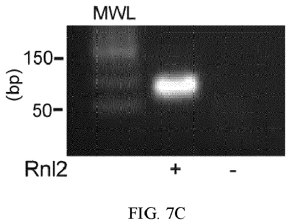

Figures 7A-7C depict a diagram, a gel, and a gel, respectively indicating that FFPE RNA could serve as a suitable template for Rnl2-mediated probe ligation in situ. Figure 7A depicts a diagram indicating the possible "on target" and "off target" sub-probe ligation events that could occur between two different probe sets. Figure 7B depicts a gel that indicates end-point PCR analysis of a forced ligation reaction showing that it is possible under unsuitable conditions to produce and readily detect off-target ligation products. Figure 7C depicts a gel that indicates the absolute requirement for Rnl2-mediated ligation in order to achieve detectable ligation product between two probes.

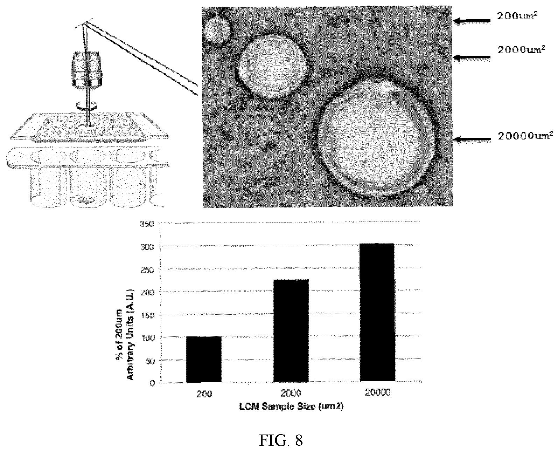

Figure 8 depicts a diagram indicating laser capture microdissection (LCM) to recover LISH product. GAPDH probe set ligation product obtained from LCM sample sizes of 200µm², 2000µm², and 20,000µm² were evaluated.

### DETAILED DESCRIPTION OF THE INVENTION

The present disclosure provides compositions and methods that are useful for performing multiplexed gene expression analysis in fixed tissue specimens such as, for example, formalin fixed paraffin embedded (FFPE). In particular, the present invention provides a method of RNA analysis that includes obtaining a sample, applying one or more multi-partite probes to the sample, where each of the one or more multi-partite probes includes at least two sub-probes, annealing at least one of the applied one or more multi-partite probes to at least one target nucleic acid within the sample, and ligating the at least two sub-probes associated with the at least one annealed multi-partite probe to create a target nucleic acid proxy that can be detected. For example, the techniques herein provide that ligation in situ hybridization ("LISH") may leverage the development of novel Rnl2-based ligation chemistry, which enables the high efficiency conversion of cross-linked RNA sequences directly into panels of gene-specific, PCR amplifiable DNA sequences, without relying on RNA extraction or reverse transcription. In another aspect, the techniques herein provide that combining LISH with next generation DNA sequencing (NGS) analysis ("LISH-seq") may enable quantitative measurement of mRNA panels in FFPE tissue specimens with unprecedented sensitivity and precision, potentially unlocking vast archives of fixed patient specimens for new analysis. LISH-seq may overcome previous limitations associated with multiplexed gene expression analysis of microdissected tissues and limiting fixed cell suspensions. Combining LISH with a unique DNA stamping and bridge PCR Amplification protocol ("LISH-stAmp") may enable spatial resolution of multiplexed gene expression analysis across an FFPE histology section or a fixed cytology sample at single cell resolution. The present disclosure is based, at least in part, on the discovery that multi-partite probes (e.g., probes including two or more sub-probes) may be annealed to a nucleic acid target (e.g., RNA) in a FFPE sample with a high degree of specificity and then ligated (e.g. by T4 RNA Ligase 2) to create a template for amplification, without requiring RNA extraction or reverse transcription. The techniques herein make use of the covalent crosslinking of RNA in fixed tissues as a beneficial feature to be exploited. Importantly, the techniques herein (e.g., LISH) are robust to degradation and adduct formation, and rely entirely on readily available reagents and equipment.

### Precision oncology

Precision oncology relies upon the ability of researchers and pathologists to molecularly characterize resected tumor tissues and biopsy materials. The development of advanced techniques for DNA analysis, such as high throughput DNA sequencing or next generation DNA sequencing (NGS), has contributed enormously to the ability to detect genetic lesions associated with cancer. However, the ability to characterize gene expression profiles in patients' fixed specimens has lagged behind, due largely to the poor quality of the RNA is such samples.

### Clinical pathogen diagnosis

The development of molecular assays that can quickly identify human pathogens remains an important goal of the diagnostic community. Tissue samples are routinely available from individuals suspected of having an as of yet unidentified fungal, bacterial, or viral infection. Yet, the extent of biomolecule degradation and processing that these samples incur during routine fixation processes renders them unsuitable for use in most currently available molecular assays. Development of alternative assays that are refractory to the detrimental fixation process, while also maintaining sensitivity, and being highly multiplex would greatly improve outcomes associated with the diagnosis and timely treatment of pathogenic infections.

Clinical tissue preservation most commonly employs a process of formalin fixation and paraffin embedding (FFPE) and FFPE samples are the most common patient specimens examined by pathologists. Their relative stability provides an ideal substrate for staining, and allows inexpensive archiving of patient tissues. Unfortunately, however, this method for preserving tissue architecture degrades and covalently modifies the tissue RNA (e.g., rendering it in a highly degraded form), hindering its analysis using traditional approaches based on RNA purification and reverse transcription. Measurement of RNA from cultured cells and unfixed tissues typically begins with RNA purification, followed by reverse transcription (RT) and polymerase chain reaction (PCR). However, RT-PCR is inefficient and unreliable when performed on RNA from chemically fixed specimens (Kashofer, K., et al. PLoS One. 8(7) (2013), Abrahamsen, HN., et al. J Mol Diagn. 5(1):34-41. (2013), Stephen. A., et al. J Biomol Tech. 15(3): 155-166. (2004)). FFPE is the most widely utilized method of preserving clinical tissue specimens, due to its maintenance of tissue architecture, biomolecule stabilization, and compatibility with a wide variety of stains, including immunostains. Formalin fixation damages RNA via addition of adducts such as hydroxyl methyl groups and by crosslinking RNA to itself and to other biomolecules via methylene bridge formation. In addition, RNA fragmentation typically occurs prior to and during tissue preservation, and then continues at lower rate during storage (Evers, D.L., et al. J Mol Diagn. 13(3):282-8. (2011), Masuda, N., Nucleic Acids Res. 27(22):4436-43. (1999)). Since RT-PCR requires relatively long stretches of minimally modified RNA, traditional techniques suffer from reduced sensitivity and unpredictable measurement biases.

Despite the importance of FFPE specimens, however, few techniques permit robust and highly multiplexed mRNA expression analysis of patients' fixed specimens, due mostly to the challenges associated with RNA extraction and reverse transcription in such samples. Existing approaches utilize either hybridization of probes without target amplification (e.g. NanoString's Prosigna assay), or targeted reverse transcription (RT) and PCR product analysis (e.g. Genomic Health's Oncotype DX qPCR assay; Assuragen's Signature LTx 12-plex fusion transcript cytometric bead array assay). Whereas the amplification-free approach is limited by its requirement for costly and proprietary instrumentation and reagents, the RT-dependent techniques are limited by the low quality of RNA (e.g., due to fragmentation, cross-linking, covalent adduct formation, etc.), thus reducing sensitivity and constraining assay design. Unbiased transcriptomic analysis using NGS is an emerging alternative, but is also limited by its requirement for RT, the high cost of unbiased sequencing, challenges associated with computational transcript quantification and the difficulty of data interpretation. Furthermore, each of these assays depends on extraction of the RNA template, which is associated with additional degradation, loss of material, and loss of spatial resolution.

### Molecular Pathology

Molecular pathology is focused on the study and diagnosis of disease through the examination of molecules within organs, tissues, or bodily fluids. Molecular pathology shares some aspects of practice with both anatomic and clinical pathology, molecular biology, biochemistry, proteomics, and genetics. A key consideration is that more accurate diagnosis is possible when the diagnosis is based on both the morphological changes of a tissue and molecular changes within the cells of the tissue. Molecular pathology is commonly used in the diagnosis of cancer and infectious diseases. Exemplary techniques include quantitative polymerase chain reaction (qPCR), multiplex PCR, DNA microarray, in situ hybridization, DNA sequencing, antibody based immunofluorescence tissue assays, molecular profiling of pathogens, and analysis of bacterial genes for antimicrobial resistance.

The emergence of molecular pathology has spawned a new era in precision medicine. Technologies to genotype and/or phenotype tumor tissues, for example, are now routinely utilized to provide accurate diagnoses and optimal therapeutic interventions. Multiplexed gene expression measurements have the potential to provide rich phenotypic information, but to date have not been widely performed on fixed tissue specimens due to limitations associated with the quality of RNA in such specimens.

### Ligation in situ Hybridization (LISH)

The techniques herein provide a powerful platform for multiplexed measurement of gene expression, which is ideally suited for analysis of fixed tissue specimens and which overcomes the critical technical limitations associated with analysis of FFPE RNA. In one embodiment, LISH utilizes the T4 RNA Ligase (Rnl2) to efficiently join sequence-specific chimeric RNA-DNA probe sets on fixed RNA target sequences *in situ.* Subsequent treatment of the tissue with RNAse H releases RNA-templated ligation product, while simultaneously destroying background, DNA-templated ligation product. As seen in Figure 5A, step-I, probes have no preference for annealing to mRNA or DNA targets which have complementary sequence. Therefore, during RNAse H treatment (step-III), two distinct pools exist, ligated-probe:RNA templates and ligated-probe:DNA templates. RNAse H specifically digest RNA of RNA:DNA duplexes. Therefore, RNAse H will recognize and digest RNA templates in ligated-probe:RNA template duplexes and conversely digest and destroy the RNA component (internal diribo) of ligated probes in ligated-probe:DNA template duplexes.

In one aspect, the techniques herein provide multi-partite nucleic acid probes that may be applied to a fixed tissue specimen (e.g. FFPE). The multi-partite probes may each include two or more nucleic acid probes (e.g., sub-probes) configured to anneal to a contiguous target sequence in a target nucleic acid (e.g., a RNA), such that each of the two or more probes bind the contiguous target sequence without leaving any unbound nucleotides in the contiguous target sequence between any of the two or more probes that are adjacent. For example, if a multipartite probe includes two probes (e.g., a bipartite probe), then once the two probes are bound to the contiguous target sequence, the 3' end of one of the probes will be immediately adjacent to the 5' end of the other probe such that the two probes are bound in and end-to-end configuration.

Once the multipartite probe is bound to the contiguous target sequence, a ligase (e.g., T4 RNA Ligase 2 (Rnl2)) may be used to ligate the adjacent, bound multi-partite probes into amplifiable cDNA counterparts, without requiring RNA extraction or relying on reverse transcription. The techniques herein (e.g., LISH) may be amenable to a highly multiplexed mRNA analysis method. For example, it is contemplated within the scope of the invention that a typical fixed specimen may be analyzed with 10-10,000 multi-partite probes simultaneously. The techniques herein, (e.g., LISH) extends previous work on RNA-templated DNA probe ligation chemistry (See e.g., Larman, H.B., et al. Nucleic acids research 42, 9146-9157 (2014)), to convert cross-linked RNA molecules into PCR-amplifiable DNA sequences. Advantageously, the techniques described herein are robust to degradation (e.g., a highly degraded sample) and adduct formation, and rely entirely on readily available reagents and equipment.

### Probe design and validation

Multi-partite probes may be designed with a probe set design pipeline similar to Primer-BLAST and implemented using Primer 3, BLASTN, Melting, pandas and the Python standard library. Probes were designed to anneal as near as possible to the target mRNA poly(A) tail. Custom Primer3 settings were implemented to design up to 20 separate 36 nucleotide probes antisense to the target transcript. Primer3-designed probes were extended 4 base pairs in the 5' direction of the probe (towards the poly(A)tail). Each 40 nucleotide sequence was then split in half and common adaptors (AD1 for acceptor probes and RCAD2 for donor probes) were appended. Primer3 was then called to calculate the properties of each probe oligo plus adaptor. Empirically derived thresholds for the Primer3 calculations were used to filter the candidate probe. Remaining probes with an off-target Tm within 10°C of the predicted on-target melting temperature were removed. A non-parametric ranking scheme using distance to poly(A) tail and Primer 3 penalty (based on the original 36 nucleotide Primer3-design probe) was then employed to select the two predict best probe sets annealing at least 10 nucleotide distance from one another for each target transcript. The acceptor oligo 3 '-terminal and 3'-penultimate bases were changed to their RNA counterparts.

Critical parameters of probe design include the probe length and the melting temperature of the annealing sequences. For a given target sequence, increasing the probe length increases the strength of the specific binding interaction, but may also increase inappropriate ligations by non-specific binding to off-target sequences and/or decrease the probe's effective concentration in the reaction.

A library of donor and acceptor probes was created to explore the impact of transcript annealing sequence length, ranging from 12 to 22 nucleotides. Junction positions were kept constant in order to eliminate potentially confounding variables such as nucleotide sequence bias. For both donor and acceptor probes, the on-target ligation yield depended on the length of the probe. Because probe cost increases with length, and because diminishing improvements were observed in relative quantification after ~20 nucleotides for most probes, a probe design algorithm was developed to identify adjacent 20 nucleotide sequences in target transcripts. However, it is contemplated within the scope of the disclosure that the length of the probes within a multi-partite probe may range from 10-200 nucleotides in length.

A probe decoy strategy was developed to reduce the sampling of transcripts expressed at very high levels to optimize the efficiency of sequence analysis. Each probe set is flanked by a common primer binding sequence, so that ligation products can be separately amplified by primers containing a short DNA barcode specific for each well. Decoy probes lack the primer binding sequences, so that they form un-amplifiable ligation products at desired levels. PCR products from multiple samples can thus be pooled together for sequencing and individual reads subsequently deconvoluted by their corresponding barcodes. The finite number of DNA sequencing reads obtained results in oversampling of highly abundant transcripts. The probe decoy strategy overcomes this sampling bias.

### Multi-partite probe

Multi-partite probes present a way of bringing together and localizing two or more nucleic acid sequences. A multi-partite probe may include a target nucleic acid binding sequence capable of hybridizing with the target genetic sequence and an additional nucleic acid binding probe sequence adjacent to the target nucleic acid binding sequence. For example, multi-partite probes may each include two or more nucleic acid probes configured to anneal to a contiguous target sequence in a target nucleic acid (e.g., a RNA), such that each of the two or more probes bind the contiguous target sequence without leaving any unbound nucleotides in the contiguous target sequence between any of the two or more probes that are adjacent (e.g., a sub-probe). A multi-partite probe may be hybridized to the genetic sample including the target genetic sequence and ligated to include a detectable amplification molecule. The detectable amplification molecule may include a capture probe sequence that is a nucleic acid sequence capable of hybridizing with the nucleic acid binding probe sequence for the bipartite probe. Each probe within the multi-partite probe may range from 10-200 bases in length. For example, 10-200 is understood to include any number, combination of numbers or sub-range of numbers, as well "nested sub ranges" that extend from either end point of the range. For example, a nested sub-range of an exemplary range of 10 to 200 may comprise 10 to 20, 10 to 30, 10 to 40, 10 to 50, 10 to 60, 10 to 70, 10 to 80, 10 to 90, 10 to 100, 10 to 110, 10 to 120, 10 to 130, 10 to 140, 10 to 150, 10 to 160, 10 to 170, 10 to 180, 10 to 190 and 10 to 200.

### Multiplexing

Multiplex gene expression analysis provides direct and quantitative measurement of multiple nucleic acid sequences simultaneously using a detection system. Multiplex assays utilize a strategy where more than one target is amplified and quantified from a single sample aliquot. In multiplex PCR, a sample aliquot is queried with multiple probes that contain fluorescent dyes in a single PCR reaction. This increases the amount of information that can be extracted from the sample. With the LSIH technique described herein, hundreds to thousands of probes can be analyzed at the same time.

### Ligase

Rnl2 (dsRNA Ligase) is an ATP-dependent dsRNA ligase that efficiency seals 3'-OH/5'PO₄ nicks in duplex RNAs. This process occurs via adenylation of the ligase, AMP transfer to the 5'PO₄ on the donor strand, and the attack by the acceptor strand 3'-OH on the 5'-adenylylated donor strand, resulting in the formation of the covalent phosphodiester linkage. Rnl2 tolerates complete substitution of its duplex RNA substrate with deoxyribonucleotides, provided that the 3'-terminus of the acceptor strands terminates in a diribonucleotide. The technique employs fully deoxyribonucleotide donor probes with 5'-PO₄ termini, which undergo highly efficient, template-dependent ligation to hybrid deoxyribonucleotide-3'-diribonucleotide acceptor probes. In summary, Rnl2 can ligate RNA probes or RNA-DNA hybrid probes in which one probe has the 3' two bases as RNA when annealed on either RNA or DNA templates. Rnl2 cannot efficiently ligate fully DNA probes annealed on DNA templates or fully DNA probes on RNA templates.

The ligase may be introduced to join adjacently annealed acceptor and donor probe sets. Enzymatic ligation covalently joins the probes, which then serves as a template for PCR-based signal amplification. Under typical conditions, all components of the ligation reaction are in excess over the target mRNA, ensuring the direct proportionality between template molecules and ligation events. Subsequently, the ligation products may be amplified and barcoded during a PCR.

It is contemplated within the scope of the disclosure that other types of ligases may be used according to the techniques herein including, but not limited to, T4 DNA ligase and PBCV-1 DNA Ligase (Chlorella virus DNA Ligase).

### RNaseH (Ribonuclease H)

Once probes are ligated, the ligated multi-partite probe (e.g., the sub-probes) may be released from the RNA template so that it may be recovered and used as an amplification source. According to the techniques herein, RNaseH may be used to release the ligated multi-partite probe (e.g., the sub-probes). RNAseH belongs to a family of non-sequence-specific endonucleases that catalyze the cleavage of RNA via a hydrolytic mechanism. Members of the RNase H family may be found in nearly all organisms, from bacteria to archaea to eukaryotes. Because RNaseH specifically degrades only the RNA in RNA:DNA hybrids, it is commonly used in molecular biology to destroy the RNA template after first-strand cDNA synthesis by reverse transcription, as well as in procedures such as nuclease protection assays. RNase H can also be used to degrade specific RNA strands when the cDNA oligonucleotide is hybridized, such as the removal of the polyadenine tail from mRNA hybridized to oligo (dT), or the destruction of a chosen non-coding RNA inside or outside the living cell. RNaseH specifically hydrolyzes the phosphodiester bonds of RNA, which is hybridized to DNA. This enzyme does not digest single or double-stranded DNA. To terminate the reaction, a chelator, such as EDTA, is often added to sequester the metal ions in the reaction mixture, or the enzyme can be heat destroyed.

Following ligation of adjacently annealed probes, the ligation product (LISH product) may be retrieved by incubation of the sample with RNAseH, which destroys the RNA component of the RNA/DNA hybrid helices and releases the ligated sub-probes into solution. The product may then be amplified and finally, sequence-analyzed. Alternative methods to recover the ligated probe sets include, but are not limited to other RNase enzymes (such as RNase A, RNase I_{f}, RNase HII, for example), thermal treatment to melt the hybrid helices or mechanical tissue extraction (e.g. by laser capture microdissection or scraping) prior to PCR amplification.

The techniques herein in have the potential to greatly expand the molecular toolbox available to the researchers and pathologists who analyze fixed tissue specimens. Current approaches to highly multiplexed gene expression analysis of FFPE tissues require extraction of damaged, relatively low quality RNA. In contrast, LISH-based analyses utilize cross-linked RNA molecules to obtain PCR amplifiable sequences, and thus does not require RNA extraction or reverse transcription, which provides several distinct advantages related to assay sensitivity and workflow simplicity. Importantly, the system utilizes widely available instrumentation and reagents, making it accessible immediately. The LISH-seq assay proposed in this application will be increasingly adopted as high throughput DNA sequencing declines in cost and grows in availability.

For applications in which spatial resolution of gene expression analysis is important, LISH-stAmp has the potential to dramatically outperform alternative assays both in terms of sensitivity and the number of transcripts that can be analyzed simultaneously. By significantly expanding the capabilities for measuring gene expression in fixed tissues, the LISH platform will greatly improve the ability to characterize tumor phenotypes and to understand the complex interactions with cells of the immune system. Finally, because the LISH platform is ideally suited to fixed tissue specimens, it will unlock the vast archives of patient samples for new, high dimensional RNA analyses that are not otherwise feasible.

### EXAMPLES

### EXAMPLE 1: Development of Ligation in situ hybridization sequencing (LISH-seq)

An optimal protocol for efficiently producing probe ligation product in FFPE sections is determined. Housekeeping gene probe sets are employed to explore the impact of various protocol parameters on the signal (on-target probe ligation) and noise (off-target probe ligation) of the assay. The performance of LISH is assessed by direct comparison with RT-qPCR. High throughput DNA sequencing of mixed LISH products ("LISH-seq") demonstrates the degree of multiplexing that the system provides (e.g., 100's to 1,000's of transcripts per tissue section). A collection of specimens stored for increasing periods of time is analyzed by LISH-seq. This dataset reveals the extent to which LISH-seq unlocks the vast archives of patient tumor specimens for highly multiplexed mRNA analyses. A strategy using LISH-seq to overcome previous limitations associated with the analysis of RNA extracted from microdissected tissues and small populations of fixed suspension cells is presented.

### RNA Annealing, Selection and Ligation Sequencing (RASL-seq) Assay

In 2012, the Fu group at UCSD introduced "RNA Annealing, Selection and Ligation sequencing" (RASL-seq), and demonstrated its ability to monitor hundreds of transcripts in thousands of samples at a lower cost and higher throughput than is currently possible with alternative approaches (See e.g., Li, H., et al. Proc Natl Acad Sci U S A 109, 4609-4614 (2012) and Zhou, Z., et al. Molecular cell 47, 422-433 (2012)).

This technical feat is accomplished by utilizing a pool of gene-specific DNA probe sets, pairs of which anneal adjacently on target transcripts. After transcript-templated enzymatic probe set ligation, PCR amplification of the ligation product is performed. The PCR primers are typically barcoded with sample-specific DNA sequences, which allows the subsequent pooling of PCR products from a large number of samples for simultaneous NGS analysis, thereby dramatically reducing the per-sample cost. A key feature of the RASL assay is that probe ligation is templated directly by short sequences of RNA, thus avoiding the requirement for reverse transcription (RT).

Previously published RASL-seq protocols relied on the T4 DNA Ligase to perform the critical ligation step. However, it has been observed that this RNA-templated ligation of DNA strands resulted in exceedingly low yield (See e.g., Table 1 in Bullard, D.R. & Bowater, R.P. The Biochemical journal 398, 135-144 (2006) Figure 1A Larman, H.B., et al. Nucleic acids research 42, 9146-9157 (2014)), and thus poor assay sensitivity.

To address this problem, as described herein, a ligation chemistry that utilizes the T4 RNA Ligase 2 (Rnl2), an enzyme that naturally repairs breaks in double-stranded RNA is developed. Importantly, Rnl2 tolerates substitution of its nicked dsRNA substrate with nicked dsDNA, provided that the 3' terminus of the acceptor strand has two RNA bases (Figure 1A) (See e.g., Nandakumar, J. & Shuman, S. Molecular cell 16, 211221 (2004)). As described in detail below, Rnl2 ligates chimeric DNA-RNA probe sets on an RNA template with quantitative yield, which is on average are 400-fold higher than the T4 DNA Ligase (Figure 1B). LISH could also be combined with additional steps to map RNA expression levels onto fixed specimens. This would allow spatial information concerning target distribution to be combined with target expression levels, a feature not possible with RASL or RT based molecular assays. Despite containing the diribonucleotide element, ligation product is PCR amplified by the multiple DNA polymerases tested. Importantly, the RASL assay is capable of measuring a spike-in transcript across a linear dynamic range of greater than six orders of magnitude (Figure 1C).

### Optimization and characterization of LISH

Human cell line derived tumor xenografts are processed into 4-40 µm thick FFPE serial sections, and are used for analysis of single housekeeping gene transcripts by means of Ligation in situ Hybridization (LISH, Figure 2A). The same protocol employed by the JHH (Johns Hopkins Hospital) clinical pathology laboratory for hybridizing RNAscope DNA probes, (See e.g., Wang, F., et al. The Journal of molecular diagnostics: JMD 14, 22-29 (2012)) currently used to detect HPV RNA in situ is used. RNAscope probe sets are highly similar to LISH probe sets, supporting this approach. Rnl2-mediated ligation follows a modified antibody staining protocol. Following extraction of ligation product, SYBR green, qPCR is used for quantification of ligation products. With a basic functional protocol in hand, the effects evaluated include:
section thickness,
pre-hybridization melting of RNA secondary structure,
LISH probe set concentration,
temperature and duration of probe set hybridization, stringency of washing,
concentration of Rnl2 ligase,
temperature and duration of the ligation step, and
the method of retrieving ligated LISH probe sets

Assay 'signal' is assessed by SYBR green qPCR analysis of correctly paired probe sets, whereas assay 'noise' is assessed by analysis of incorrectly paired probe sets. The assay signal-to-noise ratio is maximized for a series of housekeeping gene probe sets as the protocol parameters vary as described above. A LISH protocol that is compatible with traditional downstream analyses, such as immunohistochemistry is developed.

The LISH ligation product may be efficiently retrieved by using RNAseH (see e.g., step 3 in Figure 5A), which destroys the RNA component of RNA/DNA hybrid helices, to release ligated probe sets into solution. Specific areas of interest on a slide are isolated by mechanical scraping or the use of a hydrophobic barrier pen. After a brief incubation with RNAseH solution, a PCR master mix is added directly to the eluent and PCR thermocycling is initiated. For analysis of LISH probe panels using NGS, DNA barcoded primer sets are used for PCR amplification to allow sample multiplexing.

### Establishing sensitivity of LISH

The following two experiments are performed: I. Use of LISH-seq to unlock the archives of fixed tissue specimens and II. LISH-seq analysis of microdissected tissues are performed to establish the sensitivity of LISH. Archived serial sections from fixed spleen tissue is used in a LISH assay or used a serial section of equivalent size is used for the extraction of total RNA. A single tissue section is processed to extract RNA as a reference; the second serial section is processed for use in a LISH assay to compare sensitivity between the two methods. The RNA derived from FFPE sections undergoes standard single gene RT-qPCR analysis and single gene RASL assay analysis of several housekeeping gene transcripts (Figure 2B). RNA obtained from frozen unfixed human spleen tissue is used to determine the maximum signal possible from equivalent amounts of starting mRNA when used in RT-qPCR or RASL reactions. Use of the same RASL probe sets in the LISH assay provides a quantitative estimate of the fraction of RNA molecules in the fixed tissue that are converted into an amplifiable signal using the LISH protocol. Furthermore, a direct comparison between RT-qPCR and LISH analysis of fixed RNA may demonstrate the superior sensitivity of LISH-based assays.

### I. Use of LISH-seq to unlock the archives of fixed tissue specimens

Because LISH ligation products are amplified by PCR using a single primer pair, it is readily compatible with NGS sequencing (LISH-seq). Furthermore, relative gene expression patterns are well preserved even as the total amount of fixed RNA template is lost over time due to degradation. Therefore, the performance of LISH-seq analysis is assessed as a function of time that FFPE sections are stored at room temperature.

For the first series of experiments, FFPE human tumor sections previously characterized with a gene panel using RT-qPCR is used in LISH experiments to compare gene expression profiles between the different assays. Analysis of housekeeping gene mRNA and previously characterized gene panels will help establish an important framework for interpreting LISH-seq data from historical tissue specimens, and will reveal the extent to which LISH-seq unlocks the archives of fixed tissue specimens for new, highly multiplexed transcript analyses.

### II. LISH-seq analysis of microdissected tissues and fixed suspension cells.

Gene expression analyses of laser capture microdissected (LCM) fixed tissues is valuable, but remains especially challenging due to the miniscule amount, and low quality, of extractable RNA (See e.g., Pagedar, N.A., et al. Brain research 1091, 289-299 (2006) and Kaneko, T. et al. Microscopy research and technique 72, 908-912 (2009)). Performing LISH on an intact FFPE section, prior to LCM, overcomes this limitation. A protocol to PCR amplify ligation product directly from LCM material is developed and assessed using the same FFPE sections described above.

Analogous to LCM, it is extremely valuable to measure a panel of transcripts within a small population of suspension cells after FACS sorting (e.g. MHC-peptide tetramer labeled, tumor infiltrating CD4+/IFNγ + T cells). Cell fixation is typically used for staining of intracellular cytokines and transcription factors, making extraction of high quality RNA from sorted cells very difficult. A LISH protocol for fixed and permeabilized suspension cells is developed, which is also compatible with downstream analyses such as intracellular cytokine staining. Sorted cells (or possibly single cells) are then subjected directly to a PCR reaction (without nucleic acid purification) for high dimensional LISH-seq analysis.

### Alternative strategies

The protocol for annealing of LISH probe sets in FFPE tissue sections uses optimized conditions (temperatures, times) and buffers to ensure maximum stringency and sensitivity. If efficient ligation of the annealed LISH probes in situ using Rnl2 is not achieved, the activity of the Chlorella virus DNA Ligase, also known as the PBCV-1 DNA Ligase, which has recently been commercialized by NEB as "SplintR" ligase is assessed (See e.g., Lohman, G.J., et al. Nucleic Acids Res 42, 1831-1844 (2014)). In the past, the PBCV-1 Ligase and Rnl2 have been directly compared, and Rnl2 was found to perform more optimally (See e.g., Larman, H.B., et al. Nucleic acids research 42, 9146-9157 (2014)), but it is expected that the PBCV-1 Ligase function sufficiently well in LISH applications.

Additionally, nonspecific probe ligation is an undesirable reaction and can be strongly suppressed by including "decoy" probes that compete with functional probes for nonspecific ligation. Decoy probes are designed to target irrelevant sequences and they lack the PCR primer binding adapters present on the functional LISH probes. When decoy probes are added to the reaction in excess, the off target fraction of amplified ligation product may be reduced dramatically (See e.g., Larman, H.B., et al. Nucleic acids research 42, 9146-9157 (2014)).

### EXAMPLE 2: Maintaining spatial resolution of amplified LISH product: LISH-stAmp

The value of multiplexed gene expression analysis is greatly enhanced if the spatial distribution of the transcript levels is preserved. Such a technology is particularly useful for characterizing the complex interactions between tumor tissues and the cells of the immune system. A platform enabling the highly multiplexed, spatially resolved analysis of gene expression in FFPE tissue sections is transformative for both cancer researchers and pathologists.

The utility of LISH is expanded to a method to stamp and Amplify LISH product on a target surface ("LISH-stAmp") to preserve spatial information. This effort builds upon stamping DNA molecules to replicate high-density DNA microarrays. To locally amplify the stamped LISH product, bridge PCR is used, which has been described in the literature and is the foundation of Illumina's high throughput DNA sequencing technology. After defining the LISH-stAmp protocol, the techniques to analyze the bridge PCR amplification product are developed. These studies utilize both human cell line derived tumor xenograft sections and spreads from fixed suspension cell lines. The expression levels of 5-10 genes are measured simultaneously by hybridizing the bridge PCR product with multicolored fluorescent oligonucleotides. Sequential cycles of hybridization, imaging, and oligonucleotide removal are used to analyze an unprecedented number of transcripts from a single FFPE slide.

Tools to characterize the spatial architecture of tissue microenvironments are invaluable for understanding cancer pathophysiology, particularly in the context of complex tumor-immune cell interactions.

### Supramolecular nanostamping (SuNS):

Supramolecular nanostamping (SuNS) is a method developed by Francesco Stellacci and others to transfer reversibly patterned chemical information (e.g. DNA) from one surface to another (Figure 3A) (See e.g.., Yu, A.A., et al. Nano letters 5, 1061-1064 (2005), Akbulut, O., et al. Nano letters 7, 3493-3498 (2007) and Kim, J. & Crooks, R.M. Analytical chemistry 79, 7267-7274 (2007)). The SuNS methodology was extended to enzymatically `replicate' DNA microarrays the size of microscope slides (Cuppoletti, A. & Larman, H.B. (See e.g., Patent: PCT/US2008/076717, 2009)). The experiments employ the following strategy:
(i) enzymatic synthesis of a biotin-labeled cDNA strand on a ssDNA template microarray,
(ii) contacting the dsDNA microarray with a streptavidin-incorporated hydrogel film-coated capture surface,
(iii) thermal release of the biotinylated cDNA into the hydrogel where it binds streptavidin, and
(iv) separation of the slides and preparation of the template for the next replication cycle. Initial studies demonstrated both the faithful maintenance of resolution of the replica surface as well as its functionality (Figure 3B).

Herein, DNA stamping is adapted to spatially resolve gene panel expression analysis in FFPE sections.

### Experimental Design

Initial studies utilize experience in stamping DNA from a template microarray onto a hydrogel film-coated replica surface (See e.g., Cuppoletti, A. & Larman, H.B. Patent: PCT/US2008/076717, 2009) and Larman, H.B. & Stellacci, F. (See e.g., Patent: PCT/US2008/076723, 2010)). In the techniques presented herein, however, stamping DNA (LISH ligation product) from a 'hydrogel' (e.g., an FFPE tissue section) onto a replica surface coated with covalently immobilized PCR primers is performed.

### "Bridge" PCR

Bridge PCR is a method of localized, solid-phase PCR amplification, in which DNA fragments are amplified by primers attached to a surface (rather than in solution), thus forming surface-immobilized DNA clusters (See e.g., Adams, C.P. & Kron, S.J. (Patent: US 08/285,385, 1997)). If the clusters are sufficiently separated, they can be clonally sequenced (See e.g., Mitra, R.D., et al. Analytical biochemistry 320, 55-65 (2003) and Mitra, R.D., et al. Proc Natl Acad Sci USA 100, 5926-5931 (2003)) which is the basis of Illumina's high throughput DNA sequencing technology (See e.g., Shendure, J., et al. Science 309, 1728-1732 (2005) and Porreca, G.J., et al. Current protocols in molecular biology / edited by Frederick M. Ausubel ... [et al.] Chapter 7, Unit 7 8 (2006)). If the clusters are completely or partially overlapping, however, the relative contribution of each sequence is determined by hybridization with fluorescently labeled cDNA sequences, which is the basis of the LISH-stAmp technology (Figure 4).

The first series of experiments establishes the bridge PCR target surface. Epoxysilane coated glass microscope slides (Schott) are utilized, which are ideal for covalent immobilization of 5'-amino-terminated oligonucleotide primers (IDT). To assess the quality (surface density and uniformity) of the bridge PCR target surfaces, fluorescent primer-binding DNA probes are utilized.

Using target surfaces, the bridge PCR protocol for LISH product is optimized, starting with published methods. A dilute solution of pre-ligated LISH probe is spotted by hand directly onto the slide surface to mimic stamping. After bridge PCR, the slide is analyzed using a complementary hybridization probe to measure the amplification.

After successful bridge PCR using spotted ligation product is successful, experimenting with fixed tissue specimens as the source of LISH probe ligation product is initiated. The simplest method to achieve reliable, large area conformal contact between the tissue slide and the target slide is first established. A temperature-controlled press system for stamping DNA was developed (See e.g., Larman, H.B. & Stellacci, F. (Patent: PCT/US2008/076723, 2010)), and its utility for LISH-stAmp is explored. Similar results using a simple food vacuum sealer may also be achieved. Once in contact, the slides are heated to permit the thermal release of the LISH ligation product from the FFPE section, and then cooled to 45°C for sufficient time to permit capture by the bridge PCR primers on the target surface. The slides are separated and the target slide is subjected to bridge PCR amplification. Analysis of yield versus number of thermocycles is performed. FFPE sections from human cell line derived tumor xenografts is used to develop the methodology.

### LISH-stAmp studies

LISH-stAmp experiments utilize a panel of 5-10 probe sets, which are analyzed simultaneously using cDNA oligos labeled with fluorescent dyes that are discernable on the Zeiss Axiovert 200 inverted microscope (with the LSM510-Meta confocal module, seven laser lines, and four filter sets) which is housed in the Johns Hopkins Microscopy core. Studies focus primarily on two types of tissues: (i) cytospins of human cell lines, and (ii) human cell line derived tumor xenografts. Cytospin preparations permit analysis of LISH-stAmp's spatial resolution. Importantly, individual probe ligation products are spotted by hand on separate bridge PCR slides to serve as fluorescence compensation controls for the multiplexed LISH-stAmp analyses. A standard DNA microarray "stripping" protocol is employed to enable sequential probe set hybridizations, thereby allowing a much greater number of LISH probes to be analyzed than could otherwise be imaged simultaneously.

### Alternative strategies

Analysis of vastly different gene expression levels on LISH-stAmp slides may be problematic. In part, this is due to competition for bridge PCR primers between LISH products from high expression genes and LISH products from low expression genes. An analogous challenge was previously addressed in development of RASL-seq by the use of gene-specific decoy RASL probes (See e.g., Larman, H.B., et al. Nucleic acids research 42, 9146-9157 (2014)). Therefore, the effect of partially decoying housekeeping gene probe sets is expected to increase the availability of bridge PCR primers to the LISH product of lower abundance transcripts. In extremely unbalanced fluorescent signals, the signal from highly abundant transcripts is dampened by diluting the labeled hybridization probes into a background of unlabeled hybridization probes.

### EXAMPLE 3: Measurement of RNA sequences in FFPE

A recent report has demonstrated RNA-templated probe ligation chemistry that is sensitive, specific, and suitable for massively multiplexed analyses (Larman, H.B., et al. Nucleic Acids Research 42, 9146-9157 (2014), incorporated herein by reference in its entirety). The approach utilizes T4 RNA Ligase 2 (Rnl2), which ligates sequence-specific oligonucleotide probe sets annealed adjacently on an RNA "splint". In this setting, Rnl2 requires that the acceptor oligo is 3'-terminated with two ribonucleotides (here termed a "3'-diribo probe"). In some examples, the acceptor oligo may have a 3' termination of at least two RNA bases. The phosphorylated donor probe (here termed a "5'-phospho probe") can be fully DNA (FIG. 5A). The ligated 3'-diribo and 5'-phospho probe set is thus a DNA molecule that contains an internal diribonucleotide sequence.

There is no known DNA ligase that exhibits a preference for RNA versus DNA splints, and even Rnl2 ligates chimeric probe sets with roughly equal efficiency on both RNA and DNA splints. Precise quantification of RNA sequences will therefore be confounded by background signal from DNA-splinted ligation, unless the product of DNA-splinted ligation is specifically eliminated. RNAse H is a nuclease that specifically digests the RNA component of RNA-DNA hybrid helices. RNAse H therefore served a dual purpose by simultaneously (i) destroying DNA-splinted diribonucleotide-containing ligation product (FIG. 5A (i)) and (ii) releasing RNA-splinted diribonucleotide-containing ligation product from fixed RNA into solution for analysis (FIG. 5A (ii)). Indeed, DNA-annealed diribonucleotide-containing DNA sequences were cleaved by RNAse H2, whereas RNA-annealed sequences were not (FIG. 5B). RNAse H1 requires at least four contiguous bases of RNA for digestion, and so this enzyme did not destroy DNA-annealed diribonucleotide-containing DNA sequences, as expected. RNAse H2 and H1 can thus be used to recover ligated probe sets, with or without causing simultaneous destruction of DNA-splinted ligation product, respectively. This new system of measuring RNA sequences in fixed tissue is referred to as "Ligation in situ Hybridization" ("LISH").

### FFPE RNA served as a template for Rnl2-mediated probe ligation in situ

Two probe sets targeting the GAPDH andRPL19 housekeeping genes were used to assay a 10 micron section of human spleen. End-point PCR analysis of the RNAse H recovered ligation product revealed that the correct probe pairs were indeed ligated *in situ,* whereas mismatched probe sets were not ligated at detectable levels (FIGs. 5C and 5D).

To quantify the amount of specific ligation products formed during a multiplex reaction, quantitative PCR assays were performed for each of the four possible ligation products (two "on-target" and two "off-target"). These assays were then used to determine the amount of signal (on-target probe ligation) to noise (off-target probe ligation), while LISH protocol parameters were optimized

### Evaluation on clinical archives of FFPE specimens

Vast clinical archives of FFPE specimens exist around the world; a technique enabling recovery of transcriptional information from archived tissues would therefore be of extreme utility, especially to investigate limiting or high value specimens. Whereas the magnitude of recovered ligation product is expected to decline modestly as a function of time, the relative gene expression profile should be faithfully maintained using LISH analysis.

### Independent LISH assays sequentially performed on the same tissue section

Use of RNAse H to recover LISH ligation product should spare the RNA molecules that did not serve as templates for probe ligation. As a result, the untargeted RNA sequences of a previously LISH-analyzed section should remain available for a second, non-overlapping LISH analysis. This is a useful feature of LISH for investigations of limiting or high value specimens. Independent LISH assays can be performed sequentially on the same tissue section, such that the results from one LISH assay can be used to guide the selection of informative probes to be used in a second LISH assay of the same FFPE tissue section.

### LISH assay compatibility with a variety of analytical stains

A rich diversity of analytical stains are used to interrogate FFPE specimens. Many of these, including hematoxylin and eosin (H&E) and immunostains will be useful to employ in conjunction with LISH analyses. Ligated LISH probes are relatively stable, and therefore expected to remain intact *in situ* during a subsequent staining protocol. After staining, LISH product can then be retrieved using RNase H, laser capture microdissection (LCM), or other means. LISH analysis can therefore be combined with analysis of the same tissue sections using a wide variety of common stains.

### Size of microdissected tissue employed using LISH assay

Multiplexed LISH products are uniform in size and should therefore amplify with minimal PCR bias. This quality of LISH should facilitate the multiplexed analysis of extremely small fixed tissues, such as those obtained by laser capture microdissection (LCM). The smallest amount of microdissected tissue that would still provide a robust measurement of gene expression was determined. Ten micron spleen section tissue fragments that ranged in size from 20,000 square microns down to 200 square microns (the approximate equivalent of ~300 to 3 splenocyte areas, respectively) were obtained. Quantification of the housekeeping probe ligation products revealed a reliable signal, which was proportional to LCM fragment size (FIG. 8). LISH therefore performs favorably in terms of the amount of tissue required to obtain a robust multiplexed measurement of gene expression. An additional advantage is that the entire section was uniformly hybridized and ligated prior to microdissection of the desired tissue areas. This approach minimized technical variance and circumvented the costs and material losses associated with extraction of RNA from individual fragments.

### EXAMPLE 4: LISH assays for diagnosis of infection

A high sensitivity technique for detecting a large number of different RNA sequences in FFPE specimens would be extremely useful for diagnosis of infection. In clinical mycology, for example, chemical stains often reveal the presence of a fungal organism but subsequent definition of the species can be very difficult. This was the case in 2012, when a mysterious outbreak of fungal meningitis was later traced to contaminated vials of methylprednisolone.

An extremely small brain biopsy section was obtained from a patient seen at the Johns Hopkins Hospital. Multiplex LISH assays were performed using a panel of probe sets designed to distinguish among three closely related fungal species. The *Exserohilum rostratum* detector probes reported strong on-target signal from the index patient who had been infected by injection of methylprednisolone contaminated with *E*. *rostratum,* but not from biopsies of uninfected tissues or sections containing unrelated infections, such as the closely *related Aspergillus fumigatus* (FIGs. 6A-6C). LISH was therefore used to detect fungal infection using preserved clinical specimens. Highly complex probe pools could in this way be used for comprehensive diagnostic assays. In this setting, rapid analysis of ligation product could be performed using real time nanopore sequencing or mass spectroscopy, for example.

### EXAMPLE 5: LISH assays for diagnosis in immuno-oncology

Immuno-oncology is a rapidly evolving field with recent clinical trials demonstrating dramatic patient benefits (Couzin-Frankel, J. Science (2013) 342; 1432-1433). Immuno-oncology, may for example, use drugs known as immunotherapies that target the body's immune system to help fight cancer. Responses can vary widely among individuals with seemingly similar tumors, suggesting that features of the tumor microenvironment underlie, or undermine, the efficacy of an immunotherapeutic regimen. The tumor-immune interface is often a highly complex tissue architecture (e.g., structure), where numerous cell types, surface receptors, and secreted products come together to govern a patient's anti-tumor immune response. Highly multiplexed measurements of gene expression in microdissected specimens could therefore be used to stratify patient populations and guide therapeutic intervention. Importantly, the simple LISH methodology described in this example does not require specialized instrumentation or expertise, and could be immediately integrated into current clinical pathology workflows. As high throughput DNA sequencing becomes increasingly ubiquitous and inexpensive, the LISH platform and derivative techniques will be widely adopted for routine clinical and research applications.

Rnl2-based ligation of chimeric diribonucleotide-containing probes on fixed RNA will have applications beyond those presented here. For example, lymphocytes are frequently fixed and permeabilized prior to immunostaining. In one embodiment, LISH is performed prior to staining and sorting to enable gene expression in cell subpopulations, or potentially even single cells of interest. In addition, performing LISH in high throughput on fixed cell cultures might be used for multiplexed gene expression-based screening. The ability to efficiently ligate amplifiable DNA in situ on fixed RNA target sequences will also enable or facilitate diverse emerging technologies. For example, *in situ* methods for sequencing target transcripts (Lee JH et al., Science (2014); 343(6177):1360-3)., performing proximity ligation (Frei AP et al., Nature Methods (2016); 13(3): 269-75) or circularizing molecular inversion probes on fixed RNA templates (O'Roak BJ et al., Science (2012); 338(6114): 1619-22), may all benefit from the approaches described herein.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. Genbank and NCBI submissions indicated by accession number cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims.

### Summary Paragraphs

Paragraph 1. A method, comprising:
   obtaining a sample;
   applying one or more multi-partite probes to the sample, wherein each of the one or more multi-partite probes includes at least two sub-probes;
   annealing at least one of the applied one or more multi-partite probes to at least one target nucleic acid within the sample; and
   ligating the at least two sub-probes associated with the at least one annealed multi-partite probe to create a target nucleic acid proxy that can be detected.
Paragraph 2. The method of paragraph 1, further comprising:
   releasing the target nucleic acid proxy from the target nucleic acid; and
   amplifying the target nucleic acid proxy.
Paragraph 3. The method of paragraph 1, wherein the at least one target nucleic acid is RNA.
Paragraph 4. The method of paragraph 1, wherein the sub-probes comprise appended primer binding sites to facilitate subsequent amplification of the target nucleic acid proxy.
Paragraph 5. The method of paragraph 1, wherein the at least two sub-probes are ligated with an enzyme, a chemical reaction, or a photoreaction.
Paragraph 6. The method of paragraph 5, wherein the enzyme is a ligase.
Paragraph 7. The method of paragraph 6, wherein the ligase is selected from the group consisting of a T4 RNA Ligase 2 (Rnl2), T4 DNA ligase, a Chlorella virus DNA Ligase (PBCV-1 DNA Ligase), a Rnl2 derivative, PBCV-1derivative, and any combination thereof.
Paragraph 8. The method of paragraph 1, wherein each sub-probe comprises an oligonucleotide.
Paragraph 9. The method of paragraph 1, wherein at least one of the sub-probes is a DNA oligonucleotide that has a 3'-termination of at least two RNA bases.
Paragraph 10. The method of paragraph 1, wherein the at least one of the sub-probes is a DNA oligonucleotide that has a 5'-phosphorylation.
Paragraph 11. The method of paragraph 1, wherein the sample is selected from the group consisting of a cell, an organ, a tissue, and any combination thereof.
Paragraph 12. The method of paragraph 11, wherein the sample is a formalin fixed paraffin embedded (FFPE) tissue sample.
Paragraph 13. The method of paragraph 11, wherein the sample is fixed using non-formalin reagents
Paragraph 14. The method of paragraph 13, wherein the non-formalin reagents comprise glutaraldehyde, mercurial, oxidizing agents, alcohols, and picrates.
Paragraph 15. The method of paragraph 11, wherein the sample is comprised of fixed cells in suspension.
Paragraph 16. The method of paragraph 11, wherein the sample is a fixed tissue culture.
Paragraph 17. The method of paragraph 11, wherein the sample is obtained in a highly degraded form prior to fixation.
Paragraph 18. The method of paragraph 2, wherein the target nucleic acid proxy is released from the target nucleic acid by an endonuclease or recovered by denaturing the target nucleic acid proxy containing the at least two ligated sub-probes.
Paragraph 19. The method of paragraph 18, wherein the endonuclease comprises an RNase H, RNaseA, RNase If, or RNaseHIII.
Paragraph 20. The method of paragraph 19, wherein the RNase H comprises RNase H1, RNase H2, RNAse HI and RNAse HII.
Paragraph 21. The method of paragraph 2, wherein the amplified target nucleic acid proxy is analyzed using a technique selected from the group consisting of Next Generation Sequencing (NGS), Deep Sequencing, mass spectrometry based sequence or length analysis, DNA fragment sequence or length analysis by gel electrophoresis or capillary electrophoresis, and hybridization on immobilized detection probes.
Paragraph 22. The method of paragraph 12, wherein the fixed tissue specimen is processed into sections having a thickness of 1-100 microns.
Paragraph 23. The method of paragraph 1, wherein each of the at least two sub-probes of the plurality of multi-partite probes is 10-200 nucleotides in length.
Paragraph 24. The method of paragraph 1, wherein each of the at least two sub-probes of the plurality of multi-partite probes is 15-40 nucleotides in length.
Paragraph 25. The method of paragraph 1, wherein the nucleic acid proxy is amplified using PCR
Paragraph 26. The method of paragraph 25, wherein the PCR includes 10-50 cycles.
Paragraph 27. The method of paragraph 1, wherein each of the plurality of multi-partite probes includes two sub-probes.
Paragraph 28. The method of paragraph 1, wherein each of the plurality of multi-partite probes includes three sub-probes.
Paragraph 29. The method of paragraph 1, wherein said method further comprises
   re-applying one or more multi-partite probes to the sample, wherein each of the one or more multi-partite probes includes at least two sub-probes;
   annealing at least one of the applied one or more multi-partite probes to at least one target nucleic acid within the sample; and
   ligating the at least two sub-probes associated with the at least one annealed multi-partite probe to create a target nucleic acid proxy that can be detected.
Paragraph 30. The method of paragraph 1, wherein the sample is obtained from a subject suspected of suffering from or at risk of developing a viral infection, a bacterial infection, or a fungal infection.
Paragraph 31. The method of paragraph 30, wherein the target nucleic acid is selected from the group consisting of a viral nucleic acid, a bacterial nucleic acid, and a fungal nucleic acid.
Paragraph 32. The method of paragraph 31, further comprising:
   releasing the target nucleic acid proxy from the target nucleic acid;
   amplifying the target nucleic acid proxy;
   sequencing the target nucleic acid proxy, thereby identifying a viral nucleic acid, a bacterial nucleic acid or a fungal nucleic acid and diagnosing a viral infection, a bacterial infection, or a fungal infection, respectively, in said subject.
Paragraph 33. A method, comprising:
   obtaining a fixed tissue sample from a subject;
   applying one or more multi-partite probes to the fixed tissue sample, wherein each of the one or more multi-partite probes includes at least two sub-probes;
   annealing at least one of the one or more applied multi-partite probes to a target nucleic acid within the fixed tissue sample;
   ligating the at least two sub-probes associated with the at least one annealed multi-partite probe to create a target nucleic acid proxy within the fixed tissue sample; and
   stamping the ligated probes onto a replica surface coated with a plurality of immobilized PCR primers.
Paragraph 34. The method of paragraph 33, wherein the at least two sub-probes are ligated with an enzyme, a chemical reaction, or a photoreaction.
Paragraph 35. The method of paragraph 34, wherein the enzyme is a ligase.
Paragraph 36. The method of paragraph 35, wherein the ligase is selected from the group consisting of a T4 RNA Ligase 2 (Rnl2), T4 DNA Ligase, a Chlorella virus DNA Ligase (PBCV-1 DNA Ligase), a Rnl2 derivative, PBCV-1derivative, and any combination thereof.
Paragraph 37. The method of paragraph 33, wherein the sample is a formalin fixed paraffin embedded (FFPE) tissue sample.
Paragraph 38. The method of paragraph 33, wherein the target nucleic acid proxy is subjected to solid-phase amplification.
Paragraph 39. The method of paragraph 38, wherein the solid-phase amplification is Bridge PCR or rolling circle amplification (RCA).
Paragraph 40. The method of paragraph 33, wherein the surface comprises a polymer-coated glass microscope slides containing immobilized Bridge PCR primers.
Paragraph 41. The method of paragraph 33, wherein the tissue sample comprises resected tumor tissues or biopsy materials.

## Claims

1. A method, comprising:
obtaining a fixed tissue sample from a subject;
applying one or more multi-partite probes to the fixed tissue sample, wherein each of the one or more multi-partite probes includes at least two sub-probes;
annealing at least one of the one or more applied multi-partite probes to a target nucleic acid within the fixed tissue sample; and
ligating the at least two sub-probes associated with the at least one annealed multipartite probe to create a target nucleic acid proxy within the fixed tissue sample.

2. The method of claim 1 further comprising providing primers to amplify the nucleic acid proxy in situ within the fixed tissue sample.

3. The method of claim 1 or 2 further comprising amplifying the nucleic acid proxy in situ within the fixed tissue sample.

4. The method of claim 1, wherein the at least two sub-probes are ligated with an enzyme, a chemical reaction, or a photoreaction.

5. The method of claim 4, wherein the at least two sub-probes are ligated with an enzyme that is a ligase.

6. The method of claim 5, wherein the ligase is selected from the group consisting of a T4 RNA Ligase 2 (Rnl2), T4 DNA Ligase, a Chlorella virus DNA Ligase (PBCV-1 DNA Ligase), a Rnl2 derivative, PBCV-1 derivative, and any combination thereof.

7. The method of claim 1, wherein the sample is a formalin fixed paraffin embedded (FFPE) tissue sample.

8. The method of claim 3, wherein the amplification is Bridge PCR or rolling circle amplification (RCA).

9. The method of claim 1, wherein the tissue sample comprises resected tumor tissues or biopsy materials.

10. The method of claim 1, wherein the in situ amplification products are detected using in situ sequencing.

11. The method of claim 1, wherein the in situ amplification products are detected via hybridization with detection probes.
